# EUROPEAN PATENT APPLICATION

(11) **EP 4 806 779 A1**
(43) Date of publication of application: **16.09.2026**
(21) Application number: 25163521.5
(22) Date of filing: 13.03.2025
(51) Int. Cl.: B63B 59/04, B63B 59/06, B08B 17/02, B63B 79/40, C02F 1/32, F21V 9/40, F21V 23/00

(54) **ANTI-FOULING CONTROL FOR MARINE STRUCTURES**

(71) Applicant: Volvo Penta Corporation, 405 08 Göteborg (SE)
(72) Inventor: RÄFTEGÅRD, Viktor, 448 31 FLODA (SE)
(74) Representative: Ström & Gulliksson AB

(57) **Abstract**

A computer system (100; 400) for anti-fouling control for a marine structure (1), comprising processing circuitry (102; 402) configured to: obtain potential shift data (20) from a sensor (10) monitoring a control piece (5) mounted to a surface portion (6) of the marine structure (1) that is under the influence of bacterial film formation, wherein the control piece (5) comprises metal being electrically isolated from the surface portion (6); determine an ultraviolet, UV, light control action (30) based on the potential shift data (20), the UV light control action (30) comprising one or more of an intensity indicator (31) and an exposure period indicator (32); and control an UV light source (40) mounted to the marine structure (1) based on the UV light control action (30), the control causing the UV light source (40) to emit UV light (42) onto the control piece (5) and the surface portion (6).

## Description

### TECHNICAL FIELD

The disclosure relates generally to marine fouling. In particular aspects, the disclosure relates anti-fouling control for marine structures. The disclosure can be applied to marine vessels, such as leisure boats, ships, cruise ships, fishing vessels, yachts, ferries, among other vessel types. The disclosure can also be applied to other type of marine structures, such as sensor devices, camera equipment, underwater drones, oil rigs, offshore platforms, submerged cables, tidal energy devices, research submersibles, aquaculture facilities, etc. Although the disclosure may be described with respect to a particular marine structure, the disclosure is not restricted to any particular marine structure.

### BACKGROUND

Marine structures, such as marine vessels or other structures subjected to water submersion, are constantly challenged by the persistent issue of marine fouling, or biofouling. This involves the accumulation and/or propagation of both hard and soft microorganisms, plants, algae, animals, etc., on surfaces. Marine fouling affects practically any surface where bacterial activity can propagate, including metals, coated metals, plastics, rubber, composites, glass, painted surfaces, or the like. This negatively affects the operational efficiency and longevity of vessels, equipment and devices, and drives maintenance costs.

The present inventor seeks to address the issues of marine fouling and is in the present disclosure presenting one or more improvements to the art.

### SUMMARY

Traditional antifouling methods have relied heavily on toxic coatings, which, while effective, pose environmental hazards and are subject to increasing regulatory restrictions. Historically, antifouling paints contained tin-based compounds, such as tributyltin (TBT), but these were banned due to their harmful effects on marine life. Today, copper-based coatings, often using copper oxides, are commonly used as a less harmful alternative, though they still raise environmental concerns. Additionally, owners or personnel maintaining marine structures or vessels seek long-lasting solutions to reduce the need for frequent repainting.

In recent years, however, light technology based on ultraviolet light, UV, has emerged as a promising solution for antifouling control. Utilizing the germicidal properties of UV radiation, typically UV-C within the wavelength range of about 200-280 nm, it inhibits the growth and settlement of fouling organisms. Despite its potential, the application of UV technology in marine environments is presently still in its infancy and presents several challenges.

One of the primary limitations of existing UV antifouling systems is their reliance on fixed settings for light intensity and on/off cycles. These systems often lack the flexibility to adapt to the dynamic conditions of marine environments. As a result, they may either overuse energy or fail to provide adequate fouling prevention. Moreover, current systems tend to focus on measuring bioactivity in the water surrounding the surfaces, providing only indirect insights into the actual bioactivity on the surface. This approach overlooks the fact that the same concentration of bacteria can lead to vastly different levels of surface activity depending on various environmental factors, such as water temperature, geographical location, sun exposure, and water depth. These factors can influence the rate at which bacteria settle and form bacterial film (or biofilms), which can vary from as little as four hours to several weeks. Some systems attempt to use light sensors to monitor the intensity of UV reaching the surface. However, these sensors can be compromised by the formation of slime layers or more advanced fouling, which attenuates the detected light intensity and results in overestimation of the required UV dosage. Consequently, energy is often wasted, and the effectiveness of fouling prevention is reduced.

Given these challenges, there is a need for improved antifouling control approaches that can provide accurate, real-time adjustments to UV light application. Such approaches address the variability and complexity of marine environments to improve energy use, protect parts, and enhance the effectiveness of fouling prevention measures.

According to a first aspect of the disclosure, there is accordingly provided a computer system for anti-fouling control for a marine structure, the computer system comprising processing circuitry configured to obtain potential shift data from a sensor, the sensor monitoring a control piece mounted to a surface portion of the marine structure that is under the influence of bacterial film formation, wherein the control piece comprises metal being electrically isolated from the surface portion; determine an ultraviolet, UV, light control action based on the potential shift data, the UV light control action comprising one or more of an intensity indicator and an exposure period indicator; and control a UV light source mounted to the marine structure based on the UV light control action, the control causing the UV light source to emit UV light onto the control piece and the surface portion.

The first aspect of the disclosure may seek to provide a solution for monitoring and controlling bacterial film formation on marine structures. A technical benefit may include enhancing the longevity and efficiency of marine structures by effectively managing biofouling.

Optionally in some examples, including in at least one preferred example, the processing circuitry is further configured to compare the potential shift data to a threshold limit value; and in response to the potential shift data exceeding the threshold limit value, increase at least one of the intensity indicator and the exposure period indicator. A technical benefit may include ensuring responsive and effective antifouling action when bacterial activity increases.

Optionally in some examples, including in at least one preferred example, in response to the potential shift data satisfying the threshold limit value by a first measure, maintain at least one of the intensity indicator and the exposure period indicator. A technical benefit may include preventing unnecessary adjustments, improving energy use.

Optionally in some examples, including in at least one preferred example, in response to the potential shift data satisfying the threshold limit value by a second measure greater than the first measure, reduce at least one of the intensity indicator and the exposure period indicator. A technical benefit may include conserving energy while still maintaining effective control.

Optionally in some examples, including in at least one preferred example, the processing circuitry is further configured to determine a strain of said bacterial film formation; and adjust at least one of the intensity indicator and the exposure period indicator based on the strain of said bacterial film formation. A technical benefit may include allowing for targeted antifouling strategies, enhancing treatment effectiveness.

Optionally in some examples, including in at least one preferred example, the processing circuitry is further configured to obtain one or more stored UV light control actions associated with environments where the marine structure or other marine structure is or have been located at one or more previous time instances; and control the UV light source based on said obtained one or more stored UV light control actions. A technical benefit may include enabling efficient and tailored responses to known conditions.

Optionally in some examples, including in at least one preferred example, the UV light control action further comprises a light frequency mode being indicative of a pulse frequency at which the UV light source is controlled to operate. A technical benefit may include improving energy use and treatment efficacy.

Optionally in some examples, including in at least one preferred example, the UV light control action further comprises a light emission scheduling setting being indicative of a schedule for activation or deactivation of the UV light source. A technical benefit may include improving operational schedules to balance effectiveness with energy consumption.

In a second aspect there is provided a marine structure comprising the computer system of the first aspect, a sensor arranged to monitor a control piece mounted to a surface portion of the marine structure that is under the influence of bacterial film formation; the control piece comprising metal being electrically isolated from the surface portion.

The second aspect of the disclosure may seek to provide a solution for monitoring and controlling bacterial film formation on marine structures. A technical benefit may include enhancing the longevity and efficiency of marine structures by effectively managing biofouling.

Optionally in some examples, including in at least one preferred example, wherein the sensor is an electrochemical sensor. A technical benefit may include improving the precision of antifouling measures.

Optionally in some examples, including in at least one preferred example, wherein the UV light source comprises one or more of a mercury vapor lamp, UV light emitting diodes, excimer lamp, laser system, and cathode tube. A technical benefit may include offering flexibility in choosing suitable UV light technologies for different operational requirements.

Optionally in some examples, including in at least one preferred example, wherein the surface portion is coated with a material adapted to react to environmental stimuli. A technical benefit may include increasing the effectiveness of anti-fouling measures through interactive surface treatments.

In a third aspect there is provided a computer-implemented method for anti-fouling control of a marine structure, comprising obtaining, by processing circuitry of a computer system, potential shift data from a sensor, the sensor monitoring a control piece mounted to a surface portion of the marine structure that is under the influence of bacterial film formation, wherein the control piece comprises metal being electrically isolated from the surface portion; determining, by the processing circuitry, an UV light control action based on the potential shift data, the UV light control action comprising one or more of an intensity indicator and an exposure period indicator; and controlling, by the processing circuitry, an UV light source mounted to the marine structure based on the UV light control action, the control causing the UV light source to emit UV light onto the control piece and the surface portion.

The third aspect of the disclosure may seek to provide a solution for monitoring and controlling bacterial film formation on marine structures. A technical benefit may include enhancing the longevity and efficiency of marine structures by effectively managing biofouling.

In a fourth aspect there is provided a computer program product comprising program code for performing, when executed by the processing circuitry, the computer-implemented method of the third aspect.

The fourth aspect of the disclosure may seek to enable new marine structures and/or legacy marine structures to be conveniently configured, by software installation/update, to enhance their anti-fouling capabilities. A technical benefit may include enhancing the longevity and efficiency of marine structures by effectively managing biofouling.

In a fifth aspect there is provided a non-transitory computer-readable storage medium comprising instructions, which when executed by the processing circuitry, cause the processing circuitry to perform the computer-implemented method of the third aspect.

The fifth aspect of the disclosure may seek to enable new marine structures and/or legacy marine structures to be conveniently configured, by software installation/update, to enhance their anti-fouling capabilities. A technical benefit may include enhancing the longevity and efficiency of marine structures by effectively managing biofouling.

The disclosed aspects, examples (including any preferred examples), and/or accompanying claims may be suitably combined with each other as would be apparent to anyone of ordinary skill in the art. Additional features and advantages are disclosed in the following description, claims, and drawings, and in part will be readily apparent therefrom to those skilled in the art or recognized by practicing the disclosure as described herein.

There are also disclosed herein computer systems, control units, code modules, computer-implemented methods, computer readable media, and computer program products associated with the above discussed technical benefits.

### BRIEF DESCRIPTION OF THE DRAWINGS

Examples are described in more detail below with reference to the appended drawings.
**FIG. 1** is an exemplary marine structure according to an example.
**FIG. 2** is an exemplary schematic involving hardware and software that may take part in an anti-fouling control for a marine structure according to an example.
**FIG. 3** is a flowchart of an exemplary method for anti-fouling control for a marine structure according to an example.
**FIG. 4** is a schematic diagram of an exemplary computer system for implementing examples disclosed herein, according to an example.

### DETAILED DESCRIPTION

The detailed description set forth below provides information and examples of the disclosed technology with sufficient detail to enable those skilled in the art to practice the disclosure.

The present disclosure seeks to manage marine biofouling by introducing innovative approaches for antifouling control on marine structures. Unlike traditional methods with fixed settings, this solution dynamically adjusts UV light dosage based on potential shift data obtained from a sensor. This data is gathered from a control piece comprising metal, typically stainless steel, which is electrically isolated from surface portions of the structure. As bacteria settles and/or propagates on the metal of the control piece, oxidation and reduction processes occur, influencing the potential shift data. The control piece mimics bacterial activity on the surface, allowing for precise monitoring.

The sensor leverages a reference electrode, a stable and reliable component that provides accurate measurements of potential shifts. For instance, stainless steel typically shows a baseline of -150 mV/SCE (Saturated Calomel Electrode). As bacterial activity increases, this value shifts positively, indicating greater biofilm presence and potential corrosion. By using an electrically isolated metal control piece, reliable measurements are ensured, enabling accurate determination of the UV light control action that seeks to prevent bacterial film formation with a following potential ennoblement. This allows for adjustments to UV light intensity and/or exposure periods with high precision, effectively inhibiting bacterial growth while conserving energy.

These approaches provide several advantages over the prior art by allowing for real-time, adaptive control of UV light based on direct measurements of bacterial activity on marine structure surfaces. By targeting the actual biofilm formation, precise UV application is provided, enhancing both the effectiveness of bacterial inhibition and the conservation of energy resources, thereby addressing the limitations of existing antifouling technologies.

Advantageously, the described computer system can also be utilized for corrosion protection. By preventing the positive potential shift of the metal, the system can reduce the risk of corrosion. This capability allows for the use of more cost-effective materials or high-strength stainless steels that may not be as inherently corrosion-resistant. As a result, the system not only serves as an effective antifouling solution but may also enhance material longevity and reduce maintenance costs.

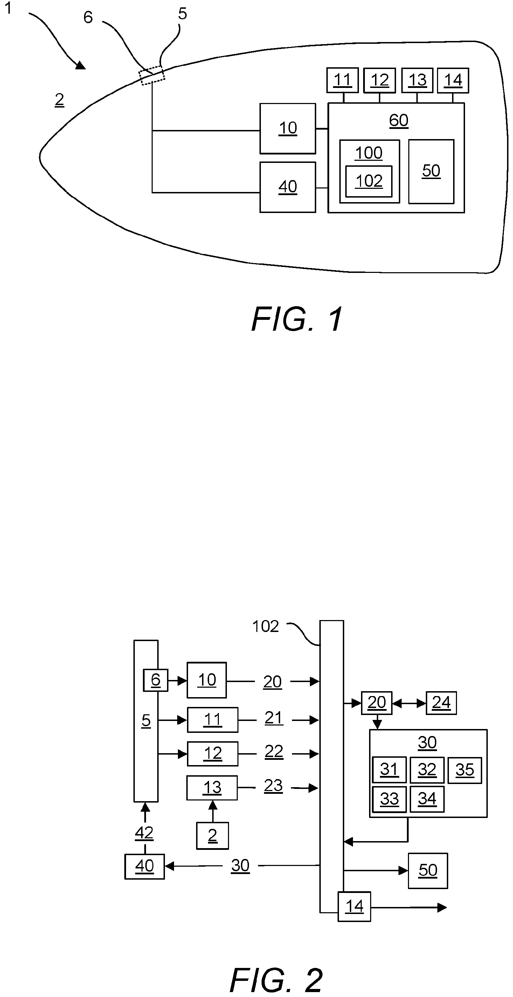

**FIG. 1** is an exemplary marine structure **1** where the inventive aspects and examples of this disclosure can be applied. The marine structure **1** may be a marine vessel **1.** The marine vessel **1** refers to any watercraft designed for navigation and operation on bodies of water. Examples include leisure boats, ships, cruise ships, fishing vessels, yachts, and ferries. These vessels operate in various aquatic environments **2,** such as oceans, seas, rivers, and lakes. While the disclosure may describe specific types of marine vessels, it is not limited to any particular kind, allowing for application across different vessel types and functions.

While not explicitly discussed for reasons of brevity, the teachings of this disclosure can likewise be applied for other marine structures **1** as well that are subjected to water submersion. Such other marine structures **1** include sensor devices, camera equipment, underwater drones, oil rigs, offshore platforms, submerged cables, tidal energy devices, research submersibles, aquaculture facilities, and much more.

The marine vessel **1** comprises at least one sensor **10.** The sensor **10** is configured to monitor a control piece **5,** enabling the obtainment of potential shift data thereof. The control piece **5** is mounted to a surface portion **6** and designed to mimic bacterial influence on the surface portion **6.** Accordingly, the surface portion **6,** and implicitly also the control piece **5** mounted thereto, are under the influence of bacterial film formation. The phrase "under the influence of bacterial film formation" as discussed herein means that the surface portion **6** and control piece **5** are experiencing colonization by microorganisms, which can lead to the establishment of a biofilm. This biofilm acts as a precursor to more severe fouling and potential corrosion, affecting integrity and performance of the material of the surface portion **6.** The role of the sensor **10** is to detect these early changes, facilitating proactive antifouling measures.

The control piece **5** can take various forms and configurations to mimic bacterial influence effectively. It may be a flat plate, cylindrical rod, or curved segment. It can be small or large, depending on the dimensions and/or size of the surface portion **6.** The size and dimension can vary, such as a few centimeters across for targeted areas or larger for broader coverage. It may have a rectangular, circular, or irregular shape. The spatial configuration might align flush with the surface or protrude slightly for enhanced exposure.

The control piece **5** can be integrated directly into the surface portion **6** or attached externally. The piece can be attached using bolts or screws for secure fastening. Adhesives or epoxy can be used for a seamless integration. Magnetic fasteners may allow easy removal for maintenance. Clips or clamps can provide temporary attachment options. Hinges might be used for adjustable positioning. Suction cups could be employed for non-permanent attachment. The control piece **5** can also be embedded within a recess on the surface portion **6.** It may use brackets for additional stability. Tethering with cables can ensure it remains aligned with the surface portion **6.** Straps or bands could offer flexible mounting options. The control piece **5** may be designed to snap into place with interlocking features. A combination of the mounting methods discussed above can be used to customize attachment based on requirements of the marine structure **1** under monitoring.

The control piece **5** comprises metal. Any metal that exhibits potential shift data can theoretically be used because these metals undergo electrochemical changes when exposed to bacterial activity, allowing the sensor **10** to detect shifts in potential. However, stainless steel is preferable due to its common use in marine environments, its well-documented electrochemical behavior, and its balance of corrosion resistance and sensitivity to biofilm formation, making it desirable for monitoring bacterial influence. The exhibition of potential shift is a direct cause of the formation and evolution of its passive film, which consists mainly of chromium oxide. When exposed to different environments, as is the case in the marine industry, this protective layer can grow, dissolve, or reform, altering the electrochemical conditions at the metal. Factors such as pH, chloride concentration, and applied potential influence the stability of this passive film, leading to shifts in the corrosion potential. Additionally, the presence of alloying elements like molybdenum or nickel can further modify the electrochemical behavior, affecting the potential shift by enhancing or weakening passivity under certain conditions.

The metal is electrically isolated from the surface portion **6.** This is to prevent interference from the other surfaces of the marine vessel **1.** Electrical isolation means that the control piece **5** is not in direct electrical contact with the surface portion **6** onto which the control piece **5** is mounted, preventing stray currents or galvanic interactions that could skew the sensor readings. This isolation may be achieved by attaching a non-conductive materials, such as insulative coatings or gaskets, to separate the metal of the control piece **5** from the material of the surface portion **6.** By isolating the control piece **5,** the obtainment of precise electrochemical data specific to the bacterial activity on the control piece **5** itself can be achieved, rather than being influenced by the overall electrical properties of the vessel **1.** This setup allows the control piece **5** to mimic the bacterial influence on the surface portion **6** while enabling reliable monitoring and analysis of potential shift data. It also makes it reliable to analyze biofouling on non-metal surface portions **6** because the control piece **5** provides consistent electrochemical data reflecting bacterial activity. This data can be correlated with biofouling patterns on non-metal surfaces, allowing for adjustments and interpretations that are applicable across different material types. By translating the measurements from the control piece **5,** insights can be applied to non-metal surfaces, enhancing the overall antifouling strategy.

Various type of sensor technologies can be employed to detect changes in electrical potential. An electrochemical sensor is typically employed, although the sensor **10** may be based on other sensor technologies, including but not limited to potentiometric sensors, voltammetric sensors, amperometric sensors, electrochemical impedance spectroscopy (EIS) sensors, etc., each capable of detecting changes in electrical potential related to bacterial activity on the control piece **5** mounted to the surface portion **6.**

The surface portion **6** can be any external surface of the marine vessel **1,** such as propeller blades, hull, rudders, thruster tunnels, stabilizers, keel surfaces, all of which are components exposed to seawater and susceptible to biofouling. The surface portion **6** can also be an internal part of the vessel **1** subjected to seawater, such as components within the seawater cooling system, including pipes, heat exchangers, or intake grates. Other internal surface portions **6** may include ballast tanks, bilge areas, internal piping systems, and the areas around the sea chest and valves, all of which are exposed to seawater to various extent, making them prone to biofouling and corrosion. The surface portion **6** may be of any type, such as relating to a flat surface, curved surface, patterned surface, or the like.

For marine structures other than vessels, the surface portion **6** can be portions of offshore platform legs, underwater pipelines, submerged cables, aquaculture nets, tidal energy turbines, wind farm foundations, bridge pillars, seawater intake screens, buoy anchors, dock pilings, marine research equipment, underwater observatories, desalination plant structures, floating solar panel supports, shipyard slipways, seawalls, port infrastructure, underwater tunnels, mooring chains, and coastal defense barriers, to name a few non-limiting examples.

In some examples, the control piece **5** and/or the surface portion **6** may be coated with a material adapted to react to environmental stimuli. The coating thus serves to enhance the detection and response capabilities. Such materials are often referred to as "smart coatings" or "responsive coatings", designed to change their physical or chemical properties in response to specific environmental triggers. Example materials for these smart coatings include conductive polymers like polyaniline or polypyrrole, hydrogel coatings like poly(N-isopropylacrylamide) or agarose gel, or photochromic materials like spiropyrans or naphthopyrans. By incorporating these smart coatings, an enhanced ability to monitor and respond to the dynamic marine environment can be provided.

The sensor **10** may be arranged at a select location at the marine vessel **1** to monitor the surface portion **6.** Multiple sensors **10** may be arranged to cover one portion or multiple portions of the hull and/or an interior surfaces of the marine vessel **1.** The sensor **10** may monitor surface portions **6** of different sizes, from small patches to extensive areas, ensuring flexibility in deployment based on specific vessel configurations. Multiple sensors **10** may be arranged in an array to gather data from a wide section of the vessel **1.** Multiple sensors **10** may be arranged strategically around the waterline of the marine vessel **1,** a region often prone to fouling due to varying water levels and exposure to sunlight. The sensor **10** may monitor surface portions **6** exposed to different environmental conditions, such as mounted to varying depths of the hull associated with different salinity levels. Multiple sensors **10** may be arranged to provide overlapping coverage, thus ensuring that no part of the vessel **1** is left unmonitored, particularly in areas known for biofouling hotspots. The sensor **10** may be arranged on surface portions **6** that are difficult to access manually, such as under hull fittings or around complex geometries, providing potential shift data without the need for frequent manual inspections. Multiple sensors **10** may be arranged to track the progression of biofouling over time, offering insights into the effectiveness of antifouling measures at different intervals and environmental conditions.

The marine vessel **1** further comprises at least one light source **40.** The light source **40** is designed to emit ultraviolet (UV) radiation, more preferably UV-C radiation, which is effective in inhibiting the growth and settlement of microorganisms responsible for biofouling. Operating within the wavelength range of about 200-280 nm, UV-C radiation disrupts the DNA and cellular functions of bacteria, algae, and other marine organisms, preventing them from colonizing the surface portions **6.**

The light source **40** is controllable, meaning it can be controlled to emit light onto the control piece **5** and the surface portion **6** based on received instructions. This control can be passive, i.e., the light source **40** operates by responding to control inputs without independently interpreting information. The control may also be active, meaning that it includes relevant control circuits, embedded firmware and/or control algorithms to process incoming data and regulate light emission. Hybrids thereof can also be envisaged.

Emitting light onto both the control piece **5** and the surface portion **6** ensures comprehensive antifouling treatment. The control piece **5** acts as a proxy, providing real-time data on bacterial activity, which is used for adjusting the light control. By also targeting the surface portion **6,** the system directly addresses biofilm formation where it occurs, enhancing the effectiveness of fouling prevention. This dual approach allows for precise adjustments to the UV light, improving energy use while ensuring both monitored and actual surfaces receive adequate treatment and that they follow each other in terms of bacterial formation build-up.

An exemplary light source **40** may include a mercury vapor lamp, which emits UV radiation and is widely used for its effectiveness and reliability in sterilization applications. Another example is UV light-emitting diodes (LEDs), which offer advantages such as energy efficiency, long lifespan, and precise wavelength control. An excimer lamp, which operates by exciting a noble gas or halogen to produce UV light, provides high-intensity and broad-spectrum UV radiation without the use of mercury. A laser system can also be used, offering focused and high-intensity UV radiation for targeted applications. A cathode tube can also be employed, emitting UV light and is a compact solution for certain configurations. Each of these technologies leverages the germicidal properties of UV radiation to effectively manage biofouling on the marine vessel **1.**

The light source **40** can be mounted to the marine vessel **1** in a variety of configurations to obtain a desirable delivery of UV radiation to the targeted control piece **5** and the surface portion **6.** It may be installed in close proximity to the sensor **10,** allowing for coordinated monitoring and light emission at specific areas prone to biofouling. Alternatively, the light source **40** can be positioned separately from the sensor, strategically placed to cover larger areas or multiple surface portions **6.**

The light source **40** is typically mounted at a calculated distance from the control piece **5** and the surface portion **6** to ensure effective UV coverage while reducing energy loss. The distance may vary depending on various factors, such as the intensity and/or type of the light source **40,** type of bacterial film foundation typically found in environments where the vessel **1** operates, energy requirements, etc., but it is generally within a range that allows for desirable exposure to the UV radiation, ensuring that the germicidal effects penetrate the biofilm effectively.

In some configurations, the light source **40** is designed to be movable, either in placement or orientation. This mobility can be achieved through one or more mechanical systems that allow the light source **40** to be repositioned along tracks, rails, adjustable mounts, or the like. This flexibility may ensure that the UV light can be directed precisely where it is needed, accommodating changes in the vessel's **1** operational conditions or biofouling patterns.

The UV light emitted by the light source **40** is associated with an intensity. The concept of intensity in the context of UV light emitted by the light source **40** refers to the strength or power of the UV radiation that is delivered to the control piece **5** and the surface portion 6. Intensity is typically measured in watts per square meter (W/m²) and determines the effectiveness of the UV treatment. Higher intensity levels result in more powerful germicidal effects, enabling the light to penetrate biofilms more effectively and deactivate microorganisms more rapidly. By adjusting the intensity of the emitted UV light, an desirable balance between effective biofilm removal, energy efficiency, and the prevention of potential surface damage or excessive energy use can be achieved.

In addition or as an alternative to intensity, the UV light emitted by the light source **40** is associated with an exposure period, being the length of time that the control piece **5** and the surface portion **6** is subjected to UV radiation. This period is may ensure that the UV light has sufficient time to disrupt the DNA and cellular processes of the fouling organisms. The duration of exposure may be determined based on factors such as the density of the biofilm, the type of organisms present, and the environmental conditions. A well balanced exposure period can ensure thorough sterilization while reducing the time the light source **40** needs to be active, thereby conserving energy.

Together, the intensity and exposure period can be coordinated to increase the antifouling effect while maintaining operational efficiency. Adjustments to these parameters can be made in real-time based on data from sensors **10** monitoring the control piece **5,** allowing for dynamic responses to changes in biofouling conditions or environmental factors. This adaptive control can ensure that the UV treatment is both effective and efficient, reducing biofouling without unnecessary energy expenditure.

In addition to intensity and exposure period, the UV light emitted by the light source **40** can also be associated with a frequency, which refers to the rate at which the light pulses. This pulsing frequency can be adjusted to improve the delivery of UV radiation based on specific fouling conditions and energy efficiency goals. The frequency of the UV light can be set to vary the intervals between pulses, allowing delivery of bursts of high-intensity radiation followed by periods of inactivity. This blinking or pulsing approach can enhance the germicidal effectiveness by intermittently shocking the biofilm with UV exposure, which can be effective in disrupting the growth cycles of microorganisms.

The marine vessel **1** may include other sensory inputs, including one or more imaging sensors **11,** biosensors **12,** and/or environment sensors **13.**

The imaging sensors **11** are used to visually monitor the surface conditions and detect the presence and extent of biofouling. These sensors can include high-resolution cameras or infrared imaging devices that capture detailed images or videos of biofilm that is accumulated on the control piece **5** and/or the surface portion **6.** The data collected can be analyzed to identify areas with biofilm accumulation or other fouling organisms. Imaging sensors **11** provide a visual confirmation of fouling levels and can be used in conjunction with other sensors to validate the effectiveness of antifouling measures.

The biosensors **12** are designed to detect specific biological markers associated with biofouling. These sensors utilize biological recognition elements, such as enzymes, antibodies, or nucleic acids, to identify and quantify the presence of microorganisms on the control piece **5** and/or the surface portion **6.** Biosensors can provide precise information about the types of organisms present and their concentration levels. This data can be used for tailoring antifouling strategies to target specific biofouling agents and for monitoring the effectiveness of UV treatment in reducing microbial activity.

The environment sensors **13** are configured to obtain data of the environment **2** where the vessel **1** is operating. The environment sensors **13** may measure parameters such as water temperature, salinity, pH, turbidity, or the like. The environment sensors **13** can also obtain geospatial data (e.g., GPS/GNSS/GLONASS data). By providing data on the environment, the environment sensors **13** enable adaptations in the operation of UV light emission based on prevailing environmental circumstances. For example, changes in water temperature and salinity can affect the rate of biofilm formation, and having this information can allow for more accurate adjustments to UV light intensity, exposure periods, and/or frequency. In another example, certain bodies of water located within a geospatial range may typically be associated with specific bacterial film formation strains.

The marine vessel **1** may further include a communication unit **14.** This would typically be a part of the vessel's **1** general communication system. Where the marine structure **1** is not a marine vessel, but other smaller and simpler devices, the communication unit **14** may be a simple transmitter capable of transferring information to a remote device, such as client device. This can make sure notifications are sent at appropriate intervals.

The marine vessel **1** may further include a graphical user interface, GUI **50.** The GUI 50 is configured to display bacterial film formation influence at the control piece **5** and/or the surface portion **6,** enabling the crew to monitor and manage the antifouling activities. The GUI **50** can be embodied in various forms, such as a touchscreen display, a computer monitor, or a panel integrated into a helm station **60** of the marine vessel **1.** The GUI **50** provides a visual representation of the potential shift data and optionally other relevant information about the control piece **5** and/or the surface portion **6,** allowing operators to easily assess the influence of bacterial film formation. The GUI **50** is designed to present data in an intuitive and accessible manner, for example using graphical elements such as charts, graphs, and color-coded indicators to convey the status of biofilm formation and the effectiveness of UV treatment. This visualization can assist the crew in quickly understanding the condition of the control piece **5** and/or the surface portion **6** and make informed decisions about antifouling measures.

In addition to displaying the potential shift data, the GUI **50** may incorporate interactive features that allow users to adjust settings, such as the intensity and exposure period of the UV light. This interactivity can ensure that operators can respond promptly to changes in biofouling conditions and affect the anti-fouling process.

The GUI **50** may also be configured to emit a visual warning when the potential shift data indicates that a threshold limit value has been exceeded. This feature acts as an alert system, drawing attention to critical situations where bacterial activity is high enough to potentially compromise the control piece **5** and/or the surface portion **6.** The visual warning can be embodied in multiple ways, such as flashing icons, color changes, or pop-up notifications on the GUI **50.**

The marine vessel **1** further comprises a computer system **100** comprising processing circuitry **102.** Generally, the computer system **100** is used for anti-fouling control of the marine vessel **1.** The computer system **100** acts as the central mechanism for collecting data from various sources, such as the sensors **10, 11, 12, 13** and/or the GUI **50,** performing one or more processing actions, and cause control of the light source **40.** The computer system **100** may be included in the helm station **60** of the marine vessel **1,** as depicted in **FIG. 1****.** Alternatively, the computer system **100** may be a system separate from the helm station **60,** for example installed elsewhere in the marine vessel **1,** in operative communication with the marine vessel **1,** or hybrids thereof. The separated system is typically used for smaller types of marine structures not being vessels. The functionality of the computer system **100** and data related therewith will now be further described with reference to **FIG. 2****.**

**FIG. 2** shows a flowchart of exemplary events taking part in an anti-fouling control of the marine vessel **1.** Orchestrating the sequence of events is the processing circuitry **102** of the computer system **100.**

The procedure begins by the processing circuitry **102** obtaining potential shift data **20** from the sensor **10.** The potential shift data refers to variations in the electrochemical potential of the control piece **5,** indicative of bacterial colonization and biofilm development. This potential shift occurs because bacterial activity on the surface alters the electrochemical environment, often leading to an ennoblement or increase in the measured potential. The control piece **5** exhibits a potential shift when immersed in seawater due to the settlement and metabolic activity of bacteria. The sensor **10** captures these electrochemical changes by measuring the voltage difference between the control piece **5** and a reference electrode. The potential shift data may be continuously collected and analyzed to monitor the condition of the control piece **5,** thereby providing real-time surveillance of the electrochemical conditions on the control piece **5.** This subsequent detection of the onset and progression of bacterial film formation, allowing for timely interventions. The potential shift data is typically measured in volts (V) or millivolts (mV), reflecting the electrical potential difference between the control piece **5** and a reference electrode. In raw format, the potential shift data is often represented as a continuous time series or discrete data points, capturing the potential at specific intervals.

In addition to the potential shift data **20,** the processing circuitry **102** may obtain one or more of image data **21,** biological markers **22,** and environmental data **23.**

The image data **21** refers to the visual information captured by the imaging sensor 11 regarding the bacterial film formation on the control piece **5** and/or the surface portion **6.** This data can include high-resolution photographs or video recordings that provide detailed views of the extent and distribution of biofouling. In raw format, the image data **21** is typically represented as digital files, such as JPEG or PNG for still images, or MPEG or AVI for video, each consisting of pixel arrays with color information that can be analyzed by the processing circuitry **102.** This data helps visually assess the severity and type of fouling and assists in validating the effectiveness of antifouling measures.

The biological markers **22** are specific biochemical indicators detected by the biosensor **12,** which are associated with the presence and activity of bacterial films on the control piece **5** and/or the surface portion **6.** The biological markers **22** can include proteins, nucleic acids, or other metabolites produced by the bacteria. In raw format, the biological markers **22** are often represented as numerical values or spectra obtained from techniques like chromatography or spectroscopy, which are recorded in data files or as part of a database. These biological markers **22** provide precise insights into the types of microorganisms present and their activity levels, allowing for targeted antifouling interventions.

The environmental data **23** encompasses various parameters measured by environment sensors **13,** such as water temperature, salinity, pH, and turbidity, which influence biofouling conditions. The environmental data **23** is typically represented in raw format as continuous time series or discrete data points stored in standard units like degrees Celsius for temperature, parts per thousand for salinity, pH units, and nephelometric turbidity units (NTU) for turbidity. The processing circuitry **102** uses this information to understand the environment **2** in which the vessel **1** operates, enabling it to adjust antifouling strategies in response to changing conditions and influence the UV light application.

Based on at least the potential shift data **20,** the processing circuitry **102** is configured to determine an UV light control action **30.** The UV light control action **30** comprises one or more of an intensity indicator **31** and an exposure period indicator **32.** In the context of the UV light control action **30,** these "indicators" refer to specific parameters or signals determined by the processing circuitry **102** that will guide the operational settings of the UV light source **40.** The intensity indicator **31** and exposure period indicator **32** are both components of this control action and serve as directives for how UV light should be applied to the control piece **5** and the surface portion **6.**

The intensity indicator **31** is a signal that specifies the required power level of the UV light to effectively inhibit biofilm formation. It acts as a directive for adjusting the output of the light source **40** based on real-time assessments of bacterial activity derived from at least the potential shift data **20.** This indicator **31** can ensure that the light intensity is appropriately modulated to match the current fouling conditions, enhancing treatment effectiveness while conserving energy. Similarly, the exposure period indicator **32** functions as a guideline for the duration the UV light should be active. It is a calculated directive that the processing circuitry **102** establishes to determine the exposure time deemed necessary to disrupt bacterial biofilms without unnecessary energy expenditure. By analyzing the potential shift data **20,** this indicator **32** ensures that the UV light remains active for the precise amount of time required to achieve effective fouling control, adapting to the changing biofouling conditions.

It should be understood that the UV light control action **30** can include either the intensity indicator **31** or the exposure period indicator **32,** but not necessarily both. In some scenarios, adjusting only the intensity of the UV light might be sufficient to address changes in bacterial activity, ensuring effective biofilm disruption with low energy use. Conversely, there may be situations where modifying the exposure period alone is adequate to maintain antifouling efficacy, particularly if the intensity is already optimized. By allowing the inclusion of one indicator without the other, the processing circuitry **102** can fine-tune its approach based on specific environmental factors and sensor data, ensuring efficient operation and tailored antifouling strategies.

In some examples, the determination of the UV light control action **30** may be based on comparisons between the potential shift data **20** and at least one predefined threshold limit value **24.** This limit value **24** represents a point where the level of bacterial activity, as indicated by the potential shift, necessitates a change in the UV light application to effectively manage biofouling. If the potential shift data **20** exceeds this limit value **24,** indicating a higher than acceptable level of bacterial film formation, the processing circuitry **102** may respond by increasing at least one of the intensity indicator **31** and the exposure period indicator **32.** The limit value **24** could be a fixed threshold limit, or a deviation from a current value of the potential shift data **20.**

In some examples, the processing circuitry **102** is configured to recognize when the potential shift data **20** satisfies the threshold limit value **24** by a first measure. This could be before the limit value **24** is reached, or after is has been exceeded and then satisfied again following a first increase in intensity and/or exposure period of UV light application. This first measure is relatively small, for example below 100 mV/SCE, indicating that the potential is close to the limit value **24.** To prevent the risk of exceeding the limit value **24,** the processing circuitry **102** maintains the current settings of the intensity and/or exposure period indicators **31, 32,** ensuring continued effective protection against biofouling without compromising the safety margin.

In some examples, the processing circuitry **102** identifies when the potential shift data satisfies the limit value **24** by a second measure, which is greater than the first measure. This indicates that the potential is comfortably within the acceptable range further from the limit value **24,** for example equal to or more than 100 mV/SCE. Given this reduced risk of exceeding the limit value **24,** the system can safely begin to decrease the intensity and/or exposure period indicators **31, 32** to conserve energy while still maintaining effective antifouling control. This approach balances energy efficiency with preventive measures.

By way of example, threshold limit values for potential shift data could involve setting specific potential levels, measured in millivolts over the electrode [mV/SCE] above which the UV light control action **30** should be modified to increase its intensity and/or exposure time. For instance, suppose the limit value **24** is set at 200 mV/SCE. When the potential exceeds this limit value **24,** indicating increased bacterial activity or biofilm formation, the processing circuitry **102** might increase the UV light intensity and/or extend the exposure period to effectively counteract the fouling. This proactive adjustment ensures that the antifouling measures remain effective across varying environmental conditions.

In some examples, multiple limit values **24** may be set, offering a layered approach to monitoring and control. These limits can represent different thresholds of bacterial activity, allowing for graduated responses as each level is reached. For instance, lower thresholds might trigger minor adjustments in UV intensity, while higher thresholds could prompt more significant changes to both intensity and exposure duration, ensuring tailored responses to varying biofouling conditions.

In some examples, the adaptations to the UV light control action **30** can be fixed, optionally between the multiple limit values **24** where those are used. This means that specific, predetermined adjustments are applied when each threshold is crossed. For example, crossing an initial limit might result in a fixed increase in light intensity, while reaching a subsequent limit could extend the exposure period by a set amount, providing structured control over the antifouling process.

In some examples, the adaptations to the UV light control action **30** can be adaptive, optionally between the multiple limit values **24** where those are used. This approach allows for dynamic adjustments based on real-time data, enabling the system to fine-tune UV light application continuously. As potential shift data fluctuates, the system can automatically modify intensity and exposure periods, improving energy use and treatment effectiveness, especially as conditions change between the set limit values.

In some examples, the adaptations to the UV light control action **30** may be exponential, increasing rapidly as bacterial activity intensifies, or logarithmic, gradually leveling off as conditions stabilize. They could also be staggered, with stepwise increases at specific intervals, or sinusoidal, oscillating based on periodic assessments of biofilm growth. Additionally, changes could be linear, with a consistent rate of adjustment, or threshold-based, activating only when certain conditions are met.

In some examples, the processing circuitry **102** can be configured to adjust at least one of the intensity indicator **31** and the exposure period indicator **32** based on a strain of the bacterial film formation. This strain refers to a specific genetic variant or subtype of a microorganism. Strains can differ significantly in their characteristics, including their growth rates, resistance to environmental factors, and susceptibility to treatments like UV radiation. In marine environments, different strains may contribute to biofouling, each potentially requiring a unique approach for effective management.

When the processing circuitry **102** adjusts the intensity indicator **31** or the exposure period indicator **32** based on the strain of the bacterial film, it is tailoring the UV light application to address the specific properties of the microorganisms present. For example, a particularly resilient strain might necessitate a higher intensity or longer exposure period to achieve effective deactivation, while a less resistant strain might be adequately managed with lower intensity or shorter exposure. By identifying the strain, and adapting either one or both of the indicators **31, 32,** the anti-fouling strategy can be improved, ensuring that the UV treatment is not only energy-efficient but also precisely targeted to the composition of the bacterial film formation.

In some examples, the processing circuitry **102** may be designed to identify the specific strain of bacterial film formation by leveraging a database of stored potential shift data corresponding to known bacterial strains. The processing circuitry **102** is configured to compare the obtained potential shift data **20** with these stored data sets. Since the potential shift data **20** reflects the electrochemical changes occurring on the control piece **5,** and consequently the surface portion **5** as a result of bacterial activity, each strain of bacteria may induce a unique electrochemical signature due to its specific metabolic processes and interactions with the stainless steel material. By maintaining a repository of potential shift data for various known bacterial strains, the processing circuitry **20** can use a comparison method, such as pattern recognition or matching algorithm, to correlate the collected data with these signatures.

When potential shift data is obtained, the processing circuitry **102** can thus analyze the data and compare it against the stored profiles. If a match or close similarity is detected, the processing circuitry **102** can accurately determine the strain of the bacterial film present on the surface. The match requirement may be determined by setting one or more match thresholds that define acceptable deviations. These thresholds can be established using statistical methods such as correlation coefficients, where a value close to 1 indicates a strong match. Alternatively, metrics like Euclidean distance may be employed, with smaller distances signifying a closer similarity. The thresholds may be calibrated based on empirical data and historical analyses to distinguish genuine strain matches from background variations or noise. This identification can allow for tailoring of the anti-fouling strategy specifically to the characteristics of the detected strain, improving the UV light control action to effectively combat the identified bacterial film formation.

In some examples, the strain can be determined by employing different methods based on different input data. The other data **21, 22, 23** discussed herein can thus also contribute to how the UV light control action **30** is determined.

In scenarios where both potential shift data **20** and imaging data **21** are utilized, the processing circuitry **102** can enhance the light control action by integrating electrochemical insights with visual assessments. This integration involves applying an image recognition method to the obtained image data **21,** which allows for automatic identification and/or quantifying of biofilm presence based on visual patterns and characteristics.

For instance, potential shift data **20** may indicate increased bacterial activity on a specific surface area, suggesting the onset of biofilm formation. Concurrently, the image recognition method processes the imaging data **21** to provide a detailed visual confirmation of biofilm density and distribution across the surface. This method involves analyzing the images to detect specific visual markers of biofilm, such as texture, color variations, and coverage patterns. By combining the quantitative potential shift data **20** with the qualitative imaging data **21,** the processing circuitry **102** can more accurately pinpoint areas requiring intensified UV treatment. This enables the processing circuitry **102** to adjust the light intensity and/or exposure period precisely, targeting the most affected regions with greater accuracy. The combined use of potential shift data **20** and image recognition can thus enhance the focus and effectiveness of the antifouling response.

In scenarios where both potential shift data **20** and biological markers **22** are utilized, the processing circuitry **102** determines the strain of bacterial film formation by comparing obtained biological markers **22** with a database of stored markers corresponding to known strains. Biological markers, which include specific proteins, nucleic acids, or metabolic byproducts, provide a biochemical fingerprint unique to each bacterial strain. By matching the obtained biological markers **22** against these stored profiles, accurate identification of the strain present on the control piece **5** and/or the surface portion **6** can be provided.

For example, the comparison may involve evaluating the biochemical signatures obtained from the biosensors **22** against established profiles in the database. The process can employ quantitative measures, such as pattern recognition algorithms or similarity indices, to assess how closely the obtained biological markers **22** align with known profiles. One or more threshold limits may be employed to define the degree of match required for accurate strain identification, ensuring that only significant similarities are considered valid.

In scenarios where both potential shift data **20** and environmental data **23** are utilized, a holistic perspective on how external conditions impact biofilm formation and growth can be obtained. The environmental sensor **13** gathers variables relating to e.g. water temperature, salinity, and turbidity, from the environment **2** where the vessel **1** is or has been located. This data is compared against a database of stored environmental data from previous locations of the same vessel **1** or other marine vessels. By analyzing these comparisons, the processing circuitry **102** discerns patterns and correlations between specific environmental conditions and biofouling rates. This comparative analysis may enable the processing circuitry **102** to fine-tune the UV light control action **30** by considering historical environmental influences on biofilm development.

For instance, if stored data indicates that similar environmental conditions previously led to accelerated bacterial growth, the processing circuitry **102** can proactively increase UV intensity and/or extend exposure periods. Such dynamic adjustments can ensure that the antifouling strategy remains robust and effective.

The environmental data **23** can thus be used to determine the strain of the bacterial film formation by correlating specific environmental conditions, such as water temperature and salinity, with known strain profiles that thrive under similar conditions. Additionally, the environmental data **23** can directly influence the UV light control action **30** by adjusting the light intensity and exposure periods in response to real-time environmental changes, such as increasing UV intensity during periods of high nutrient availability, which accelerates biofilm growth.

When all four data types-potential shift data 20, imaging data **21**, biological markers **22,** and environmental data **23**-are integrated, the processing circuitry **102** can provide a detailed and comprehensive UV light control action **30**. This holistic method may enable correlation of electrochemical changes via the potential shift data **20,** visual assessments of biofilm density via the imaging data **21,** microbial composition through the biological markers **22,** and environmental data **23** from current and historical records. Such a multi-layered antifouling strategy allows for precise and adaptive responses. For instance, if potential shift data **20** and imaging data **21** reveal significant fouling in warm, nutrient-rich waters as determined by the environmental data **23,** and biological markers **22** identify a particularly virulent strain, the processing circuitry **102** could increase UV intensity and/or adjust exposure periods to target the biofilm more effectively.

In some examples, the processing circuitry **102** is configured to determine a positioning indicator **33** as part of the UV light control action **30.** The positioning indicator **33** specifies the location and orientation of the control piece **5** and/or the surface portion **6** that requires UV treatment. By incorporating this positioning information, the processing circuitry **102** can direct the control of the UV light source **40,** causing it to adjust its position and/or orientation to obtained higher exposure of the targeted area. This may be useful for managing complex geometries or surface portions **6** that are not easily accessible. The positioning indicator **33** enables the processing circuitry **102** to dynamically reposition the light source **40,** potentially using the devices discussed above (i.e., the tracks, rails, adjustable mounts, or the like) to maintain a more precise alignment with the area of interest.

In some examples, the processing circuitry **102** is configured to determine a light frequency mode **34** as part of the UV light control action **30.** The light frequency mode **34** dictates the pulse frequency at which the UV light source **40** operates. The light frequency mode **34** may allow the processing circuitry **102** to modulate the light in a pulsing manner, delivering bursts of UV radiation at controlled intervals rather than continuous exposure. For instance, higher pulse frequencies might be employed to disrupt particularly resilient biofilms more effectively, while lower frequencies could be used to conserve energy during periods of reduced fouling activity.

It shall be noted that either one or both of the positioning indicator **33** and the light frequency mode **34** can be based on not only the potential shift data **22,** but also on the other data **21, 22, 23** discussed herein.

Following the determination of the light control action **30,** the processing circuitry **102** is configured to transmit the light control action **30** to the light source **40** for subsequent emission of UV light **42** onto the control piece **5** and the surface portion **6.** The way the UV light **42** is emitted thus depends on what instructions are included in the light control action **30** based on teachings discussed herein.

As is seen in **FIG. 2****,** the anti-fouling control is operates in a feedback loop, meaning it continuously cycles through a process of monitoring, adjusting, and reassessing. Once the light control action **30** has been executed by the light source **40** and the UV light **42** has been emitted, the sensor **10** collects additional potential shift data **20** to evaluate the impact of the UV light application. This new data informs subsequent determinations, allowing the processing circuitry **102** to refine the light control action until the bacterial film formation no longer poses a risk. The process may continue, adjusting the UV light parameters, until the potential shift data **20** satisfies the established limit values **24,** ensuring effective and efficient antifouling control.

The GUI **50** may be part of the feedback loop, receiving updates throughout the cycle. As the sensor **10** collects new potential shift data **20** and the processing circuitry **102** adjusts the UV light control action **30,** the GUI **50** may display real-time information about the process. This includes updates on current bacterial activity levels, UV light adjustments, and the whether threshold limit value(s) **24** has/have been satisfied. By providing this ongoing feedback, the GUI **50** enables operators to monitor the procedure and make informed decisions.

In some examples, the communication unit **14** as discussed above is instructed to communicate results of the feedback loop to a remote device.

In some examples, the processing circuitry **102** can obtain stored UV light control actions that are associated with specific environments where the marine vessel **1** or other similar structures have been located in the past. This capability allows for the geo-tagging of positions, enabling storage and recollection of specific settings tailored to particular locations. When the marine vessel **1** revisits a previously tagged location, the processing circuitry **102** can retrieve the stored control actions that were effective in that environment. These actions may include adjustments to UV light intensity, exposure periods, or other relevant parameters that were selected for those specific conditions. By using historical data, the system can proactively adjust the UV light source **40** to suit the environmental characteristics of the location, such as water temperature, salinity, or biofouling patterns.

In some examples, one or more auxiliary control pieces can be analyzed by the processing circuitry **102.** These auxiliary control pieces are not exposed to the same UV light emission as the primary control piece **5.** These auxiliary control pieces function to enhance the understanding of bacterial activity, enabling more precise adjustments and control of the light control actions. By being positioned in locations similar to the primary control piece and designed in a comparable manner, these auxiliary pieces provide comparative data on bacterial growth and biofilm formation. The lack of UV exposure on these auxiliary control pieces allows them to serve as baseline references, reflecting natural bacterial activity without the influence of UV treatment. This comparative approach can enable the processing circuitry **102** to discern the effectiveness of UV light adjustments more accurately, as it can compare the changes in bacterial activity between treated and untreated surfaces. By analyzing data from both the primary control piece **5** and auxiliary control piece(s), the processing circuitry **102** may fine-tune the intensity and/or exposure period, and optionally other parameter(s) of the UV light control action.

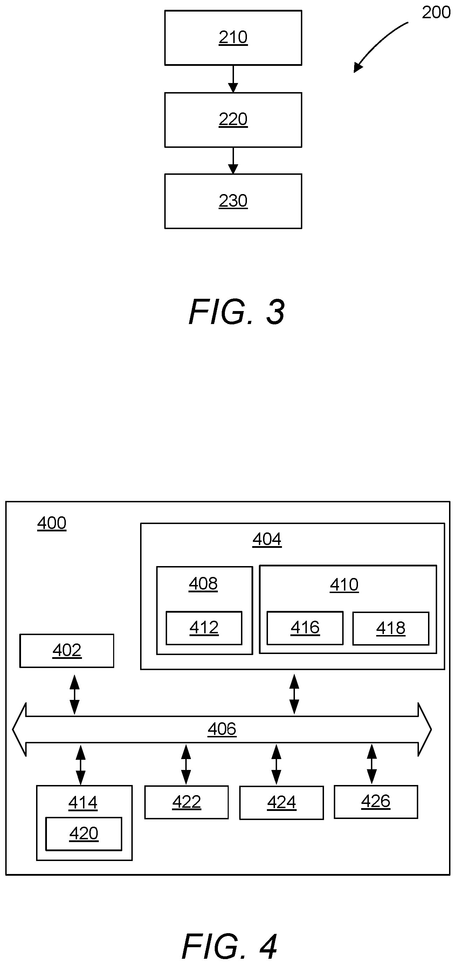

**FIG. 3** is a flowchart of a computer-implemented method **200** for anti-fouling control of a marine vessel **1.** The steps of the method **200** are carried out by the processing circuitry **102** of the computer system **100.** The method **200** comprises a step **210** of obtaining potential shift data **20** from a sensor **10.** The sensor **10** monitors a control piece **5** mounted to a surface portion **6** of the marine structure **1** that is under the influence of bacterial film formation. The control piece **5** comprises metal being electrically isolated from the surface portion **6.** The method **200** comprises a step **220** of determining a UV light control action **30** based on the potential shift data **20.** The UV light control action **30** comprises one or more of an intensity indicator **31** and an exposure period indicator **32.** The method **200** comprises a step **230** of controlling an UV light source **40** mounted to the marine vessel **1** based on the UV light control action **30.** The control causes the UV light source to emit UV light **42** onto the control piece **5** and the surface portion **6.**

**FIG. 4** is a schematic diagram of a computer system **400** for implementing examples disclosed herein. The computer system **400** is adapted to execute instructions from a computer-readable medium to perform these and/or any of the functions or processing described herein. The computer system **400** may be connected (e.g., networked) to other machines in a LAN (Local Area Network), LIN (Local Interconnect Network), automotive network communication protocol (e.g., FlexRay), an intranet, an extranet, or the Internet. While only a single device is illustrated, the computer system **400** may include any collection of devices that individually or jointly execute a set (or multiple sets) of instructions to perform any one or more of the methodologies discussed herein. Accordingly, any reference in the disclosure and/or claims to a computer system, computing system, computer device, computing device, control system, control unit, electronic control unit (ECU), processor device, processing circuitry, etc., includes reference to one or more such devices to individually or jointly execute a set (or multiple sets) of instructions to perform any one or more of the methodologies discussed herein. For example, control system may include a single control unit or a plurality of control units connected or otherwise communicatively coupled to each other, such that any performed function may be distributed between the control units as desired. Further, such devices may communicate with each other or other devices by various system architectures, such as directly or via a Controller Area Network (CAN) bus, etc.

The computer system **400** may comprise at least one computing device or electronic device capable of including firmware, hardware, and/or executing software instructions to implement the functionality described herein. The computer system **400** may include processing circuitry **402** (e.g., processing circuitry including one or more processor devices or control units), a memory **404,** and a system bus **406.** The computer system **400** may include at least one computing device having the processing circuitry **402.** The system bus **406** provides an interface for system components including, but not limited to, the memory **404** and the processing circuitry **402.** The processing circuitry **402** may include any number of hardware components for conducting data or signal processing or for executing computer code stored in memory **404.** The processing circuitry **402** may, for example, include a general-purpose processor, an application specific processor, a Digital Signal Processor (DSP), an Application Specific Integrated Circuit (ASIC), a Field Programmable Gate Array (FPGA), a circuit containing processing components, a group of distributed processing components, a group of distributed computers configured for processing, or other programmable logic device, discrete gate or transistor logic, discrete hardware components, or any combination thereof designed to perform the functions described herein. The processing circuitry **402** may further include computer executable code that controls operation of the programmable device.

The system bus **406** may be any of several types of bus structures that may further interconnect to a memory bus (with or without a memory controller), a peripheral bus, and/or a local bus using any of a variety of bus architectures. The memory **404** may be one or more devices for storing data and/or computer code for completing or facilitating methods described herein. The memory **404** may include database components, object code components, script components, or other types of information structure for supporting the various activities herein. Any distributed or local memory device may be utilized with the systems and methods of this description. The memory **404** may be communicably connected to the processing circuitry **402** (e.g., via a circuit or any other wired, wireless, or network connection) and may include computer code for executing one or more processes described herein. The memory **404** may include non-volatile memory **408** (e.g., read-only memory (ROM), erasable programmable read-only memory (EPROM), electrically erasable programmable read-only memory (EEPROM), etc.), and volatile memory **410** (e.g., random-access memory (RAM)), or any other medium which can be used to carry or store desired program code in the form of machine-executable instructions or data structures and which can be accessed by a computer or other machine with processing circuitry **402.** A basic input/output system (BIOS) **412** may be stored in the non-volatile memory **408** and can include the basic routines that help to transfer information between elements within the computer system **400.**

The computer system **400** may further include or be coupled to a non-transitory computer-readable storage medium such as the storage device **414,** which may comprise, for example, an internal or external hard disk drive (HDD) (e.g., enhanced integrated drive electronics (EIDE) or serial advanced technology attachment (SATA)), HDD (e.g., EIDE or SATA) for storage, flash memory, or the like. The storage device **414** and other drives associated with computer-readable media and computer-usable media may provide non-volatile storage of data, data structures, computer-executable instructions, and the like.

Computer-code which is hard or soft coded may be provided in the form of one or more modules. The module(s) can be implemented as software and/or hard-coded in circuitry to implement the functionality described herein in whole or in part. The modules may be stored in the storage device **414** and/or in the volatile memory **410,** which may include an operating system **416** and/or one or more program modules **418.** All or a portion of the examples disclosed herein may be implemented as a computer program **420** stored on a transitory or non-transitory computer-usable or computer-readable storage medium (e.g., single medium or multiple media), such as the storage device **414,** which includes complex programming instructions (e.g., complex computer-readable program code) to cause the processing circuitry **402** to carry out actions described herein. Thus, the computer-readable program code of the computer program **420** can comprise software instructions for implementing the functionality of the examples described herein when executed by the processing circuitry **402.** In some examples, the storage device **414** may be a computer program product (e.g., readable storage medium) storing the computer program **420** thereon, where at least a portion of a computer program **420** may be loadable (e.g., into a processor) for implementing the functionality of the examples described herein when executed by the processing circuitry **402.** The processing circuitry **402** may serve as a controller or control system for the computer system **400** that is to implement the functionality described herein.

The computer system **400** may include an input device interface **422** configured to receive input and selections to be communicated to the computer system **400** when executing instructions, such as from a keyboard, mouse, touch-sensitive surface, etc. Such input devices may be connected to the processing circuitry **402** through the input device
interface **422** coupled to the system bus **406** but can be connected through other interfaces, such as a parallel port, an Institute of Electrical and Electronic Engineers (IEEE) 1394 serial port, a Universal Serial Bus (USB) port, an IR interface, and the like. The computer system **400** may include an output device interface **424** configured to forward output, such as to a display, a video display unit (e.g., a liquid crystal display (LCD) or a cathode ray tube (CRT)). The computer system **400** may include a communications interface **426** suitable for communicating with a network as appropriate or desired.

The operational actions described in any of the exemplary aspects herein are described to provide examples and discussion. The actions may be performed by hardware components, may be embodied in machine-executable instructions to cause a processor to perform the actions, or may be performed by a combination of hardware and software. Although a specific order of method actions may be shown or described, the order of the actions may differ. In addition, two or more actions may be performed concurrently or with partial concurrence.

In further examples of the disclosure the following examples are provided.

Example 1: A computer system for anti-fouling control for a marine structure, the computer system comprising processing circuitry configured to obtain potential shift data from a sensor, the sensor monitoring a control piece mounted to a surface portion of the marine structure that is under the influence of bacterial film formation, wherein the control piece comprises metal being electrically isolated from the surface portion; determine an ultraviolet, UV, light control action based on the potential shift data, the UV light control action comprising one or more of an intensity indicator and an exposure period indicator; and control a UV light source mounted to the marine structure based on the UV light control action, the control causing the UV light source to emit UV light onto the control piece and the surface portion.

Example 2: The computer system of Example 1, wherein the processing circuitry is further configured to compare the potential shift data to a threshold limit value; and in response to the potential shift data exceeding the threshold limit value, increase at least one of the intensity indicator and the exposure period indicator.

Example 3: The computer system of Example 2, wherein the processing circuitry is further configured to, in response to the potential shift data satisfying the threshold limit value by a first measure, maintain at least one of the intensity indicator and the exposure period indicator.

Example 4: The computer system of Example 3, wherein the processing circuitry is further configured to, in response to the potential shift data satisfying the threshold limit value by a second measure greater than the first measure, reduce at least one of the intensity indicator and the exposure period indicator.

Example 5: The computer system of any preceding example, wherein the processing circuitry is further configured to determine a strain of said bacterial film formation; and adjust at least one of the intensity indicator and the exposure period indicator based on the strain of said bacterial film formation.

Example 6: The computer system of Example 5, wherein the processing circuitry is configured to determine the strain of said bacterial film formation by comparing the potential shift data with stored potential shift data for at least one known bacterial film formation strain.

Example 7: The computer system of any of Examples 5-6, wherein the processing circuitry is configured to determine the strain of said bacterial film formation by obtaining image data of the bacterial film formation from an imaging sensor; and apply an image recognition method to said image data.

Example 8: The computer system of any of Examples 5-7, wherein the processing circuitry is configured to determine the strain of said bacterial film formation by obtaining one or more biological markers of said bacterial film formation from a biosensor; and comparing said one or more biological markers with stored biological markers for at least one known bacterial film formation strain.

Example 9: The computer system of any of Examples 5-8, wherein the processing circuitry is configured to determine the strain of said bacterial film formation by obtaining environmental data of an environment where the marine structure is or has been located, the environmental data being obtained from an environment sensor; and comparing said environmental data with stored environmental data of environments where the marine structure or other marine structure is or have been located at one or more previous time instances.

Example 10: The computer system of any preceding example, wherein the processing circuitry is further configured to obtain one or more stored UV light control actions associated with environments where the marine structure or other marine structure is or have been located at one or more previous time instances; and control the UV light source based on said obtained one or more stored UV light control actions.

Example 11: The computer system of any preceding example, wherein the UV light control action includes a positioning indicator of the surface portion, wherein the processing circuitry is configured to adjust one or more of a position and an orientation of the UV light source based on the positioning indicator.

Example 12: The computer system of any preceding example, wherein the UV light control action further comprises a light frequency mode being indicative of a pulse frequency at which the UV light source is controlled to operate.

Example 13: The computer system of any preceding example, wherein the UV light control action further comprises a light emission scheduling setting being indicative of a schedule for activation or deactivation of the UV light source.

Example 14: The computer system of any preceding example, wherein the processing circuitry is further configured to obtain environmental data of an environment where the marine structure is or have been located, the environmental data being obtained from an environment sensor; and determine the UV light control action based on the environmental data.

Example 15: A marine structure, comprising the computer system of any of Examples 1-14; a sensor arranged to monitor a control piece mounted to a surface portion of the marine structure that is under the influence of bacterial film formation; the control piece comprising metal being electrically isolated from the surface portion; and a UV light source mounted to the marine structure.

Example 16: The marine structure of Example 15, wherein the sensor is an electrochemical sensor.

Example 17: The marine structure of any of Examples 15-16, wherein the UV light source comprises one or more of a mercury vapor lamp, UV light emitting diodes, excimer lamp, laser system, and cathode tube.

Example 18: The marine structure of any of Examples 15-17, wherein the surface portion is coated with a material adapted to react to environmental stimuli.

Example 19: The marine structure of any of Examples 15-18, further comprising an imaging sensor configured to obtain image data of the bacterial film formation.

Example 20: The marine structure of any of Examples 15-19, further comprising a biosensor configured to obtain one or more biological markers of the bacterial film formation.

Example 21: The marine structure of any of Examples 15-20, further comprising an environment sensor configured to obtain environmental data of an environment where the marine structure is or have been located.

Example 22: The marine structure of any of Examples 15-21, further comprising a communication unit configured to communicate feedback of the control of the UV light control action to a remote device.

Example 23: The marine structure of any of Examples 15-22, wherein the marine structure is a marine vessel, and wherein the marine vessel further comprises a graphical user interface, GUI, configured to display bacterial film formation influence at the surface portion over time based on the control caused by the computer system.

Example 24: The marine structure of Example 23, wherein the GUI is configured to emit a visual warning in response to potential shift data pertaining to said bacterial film formation influence exceeds a threshold limit value.

Example 25: A computer-implemented method for anti-fouling control of a marine structure, comprising obtaining, by processing circuitry of a computer system, potential shift data from a sensor, the sensor monitoring a control piece mounted to a surface portion of the marine structure that is under the influence of bacterial film formation, wherein the control piece comprises metal being electrically isolated from the surface portion; determining, by the processing circuitry, an UV light control action based on the potential shift data, the UV light control action comprising one or more of an intensity indicator and an exposure period indicator; and controlling, by the processing circuitry, an UV light source mounted to the marine structure based on the UV light control action, the control causing the UV light source to emit UV light onto the control piece and the surface portion.

Example 26: A computer program product comprising program code for performing, when executed by the processing circuitry, the computer-implemented method of Example 25.

Example 27: A non-transitory computer-readable storage medium comprising instructions, which when executed by the processing circuitry, cause the processing circuitry to perform the computer-implemented method of Example 25.

The terminology used herein is for the purpose of describing particular aspects only and is not intended to be limiting of the disclosure. As used herein, the singular forms "a," "an," and "the" are intended to include the plural forms as well, unless the context clearly indicates otherwise. As used herein, the term "and/or" includes any and all combinations of one or more of the associated listed items. It will be further understood that the terms "comprises," "comprising," "includes," and/or "including" when used herein specify the presence of stated features, integers, actions, steps, operations, elements, and/or components, but do not preclude the presence or addition of one or more other features, integers, actions, steps, operations, elements, components, and/or groups thereof.

It will be understood that, although the terms first, second, etc., may be used herein to describe various elements, these elements should not be limited by these terms. These terms are only used to distinguish one element from another. For example, a first element could be termed a second element, and, similarly, a second element could be termed a first element without departing from the scope of the present disclosure.

Relative terms such as "below" or "above" or "upper" or "lower" or "horizontal" or "vertical" may be used herein to describe a relationship of one element to another element as illustrated in the Figures. It will be understood that these terms and those discussed above are intended to encompass different orientations of the device in addition to the orientation depicted in the Figures. It will be understood that when an element is referred to as being "connected" or "coupled" to another element, it can be directly connected or coupled to the other element, or intervening elements may be present. In contrast, when an element is referred to as being "directly connected" or "directly coupled" to another element, there are no intervening elements present.

Unless otherwise defined, all terms (including technical and scientific terms) used herein have the same meaning as commonly understood by one of ordinary skill in the art to which this disclosure belongs. It will be further understood that terms used herein should be interpreted as having a meaning consistent with their meaning in the context of this specification and the relevant art and will not be interpreted in an idealized or overly formal sense unless expressly so defined herein.

It is to be understood that the present disclosure is not limited to the aspects described above and illustrated in the drawings; rather, the skilled person will recognize that many changes and modifications may be made within the scope of the present disclosure and appended claims. In the drawings and specification, there have been disclosed aspects for purposes of illustration only and not for purposes of limitation, the scope of the disclosure being set forth in the following claims.

## Claims

1. A computer system (100; 400) for anti-fouling control for a marine structure (1), the computer system (100; 400) comprising processing circuitry (102; 402) configured to:
obtain potential shift data (20) from a sensor (10), the sensor (10) monitoring a control piece (5) mounted to a surface portion (6) of the marine structure (1) that is under the influence of bacterial film formation, wherein the control piece (5) comprises metal being electrically isolated from the surface portion (6);
determine an ultraviolet, UV, light control action (30) based on the potential shift data (20), the UV light control action (30) comprising one or more of an intensity indicator (31) and an exposure period indicator (32); and
control an UV light source (40) mounted to the marine structure (1) based on the UV light control action (30), the control causing the UV light source (40) to emit UV light (42) onto the control piece (5) and the surface portion (6).

2. The computer system (100; 400) of claim 1, wherein the processing circuitry (102; 402) is further configured to:
compare the potential shift data (20) to a threshold limit value (24); and
in response to the potential shift data (20) exceeding the threshold limit value (24), increase at least one of the intensity indicator (31) and the exposure period indicator (32).

3. The computer system (100; 400) of claim 2, wherein the processing circuitry (102; 402) is further configured to:
in response to the potential shift data (20) satisfying the threshold limit value (24) by a first measure, maintain at least one of the intensity indicator (31) and the exposure period indicator (32).

4. The computer system (100; 400) of claim 3, wherein the processing circuitry (102; 402) is further configured to:
in response to the potential shift data (20) satisfying the threshold limit value (24) by a second measure greater than the first measure, reduce at least one of the intensity indicator (31) and the exposure period indicator (32).

5. The computer system (100; 400) of any preceding claim, wherein the processing circuitry (102; 402) is further configured to:
determine a strain of said bacterial film formation; and
adjust at least one of the intensity indicator (31) and the exposure period indicator (32) based on the strain of said bacterial film formation.

6. The computer system (100; 400) of any preceding claim, wherein the processing circuitry (102; 402) is further configured to:
obtain one or more stored UV light control actions associated with environments where the marine structure (1) or other marine structure is or have been located at one or more previous time instances; and
control the UV light source (40) based on said obtained one or more stored UV light control actions.

7. The computer system (100; 400) of any preceding claim, wherein the UV light control action (30) further comprises a light frequency mode (34) being indicative of a pulse frequency at which the UV light source (40) is controlled to operate.

8. The computer system (100; 400) of any preceding claim, wherein the UV light control action (30) further comprises a light emission scheduling setting (35) being indicative of a schedule for activation or deactivation of the UV light source (40).

9. A marine structure (1), comprising:
the computer system (100; 400) of any of claims 1-8;
a sensor (10) arranged to monitor a control piece (5) mounted to a surface portion (6) of the marine structure (1) that is under the influence of bacterial film formation;
the control piece (5) comprising metal being electrically isolated from the surface portion (6); and
an UV light source (40) mounted to the marine structure (1).

10. The marine structure (1) of claim 9, wherein the sensor (10) is an electrochemical sensor.

11. The marine structure (1) of any of claims 9-10, wherein the UV light source (40) comprises one or more of a mercury vapor lamp, UV light emitting diodes, excimer lamp, laser system, and cathode tube.

12. The marine structure (1) of any of claims 9-11, wherein the surface portion (6) is coated with a material adapted to react to environmental stimuli.

13. A computer-implemented method (200) for anti-fouling control of a marine structure (1), comprising:
obtaining (210), by processing circuitry (102; 402) of a computer system (100; 400), potential shift data (20) from a sensor (10), the sensor (10) monitoring a control piece (5) mounted to a surface portion (6) of the marine structure (1) that is under the influence of bacterial film formation, wherein the control piece (5) comprises metal being electrically isolated from the surface portion (6);
determining (220), by the processing circuitry (102; 402), an UV light control action (30) based on the potential shift data (20), the UV light control action (30) comprising one or more of an intensity indicator (31) and an exposure period indicator (32); and
controlling (230), by the processing circuitry (102; 402), an UV light source (40) mounted to the marine structure (1) based on the UV light control action (30), the control causing the UV light source (40) to emit UV light (42) onto the control piece (5) and the surface portion (6).

14. A computer program product comprising program code for performing, when executed by the processing circuitry (102; 402), the computer-implemented method (200) of claim 13.

15. A non-transitory computer-readable storage medium comprising instructions, which when executed by the processing circuitry (102; 402), cause the processing circuitry (102; 402) to perform the computer-implemented method (200) of claim 13.
